# EUROPEAN PATENT SPECIFICATION

(11) **EP 2 540 892 B1**
(45) Date of publication and mention of the grant of the patent: **16.04.2014**
(21) Application number: 11172330.0
(22) Date of filing: 01.07.2011
(51) Int. Cl.: D04H 5/02, D04H 5/06, D04H 13/00, A47L 13/17, A61K 8/02

(54) **Water dispersible nonwoven fabric material**
Wasserdispergierbares Vliesstoffmaterial
Tissu non tissé dispersible dans l'eau

(43) Date of publication of application: 02.01.2013
(73) Proprietor: Suominen Corporation, 00180 Helsinki (FI)
(72) Inventor: Pedoja, Roberto, I-22076 Mozzate (IT)
(74) Representative: Long, Giorgio

(56) References cited:
- WO-A1-2006/011167
- WO-A1-2011/019895
- WO-A1-2011/046478
- WO-A2-98/29590
- WO-A2-2009/010942
- WO-A2-2011/009997
- US-A1- 2005 106 223

## Description

The present invention relates to the field of nonwoven textile products and is applied to the manufacture of nonwoven fabric, particularly general nonwoven products, for various applications such as household use, personal and hygienic care. In particular, the present invention is applied to the manufacture of wipes and cleaning cloths.

Wet or impregnated wipes are of wide application in several fields. For example, nonwoven fabric cloths are used for cleaning purposes and may be impregnated with waxes or other cleaning solutions. Wet wipes are also used for personal care and may contain detergents, perfumes or even cosmetic lotions or creams.

These products are normally made from cellulose-based raw materials (100% cellulose or in any case a high cellulose content), such as viscose, cotton and the like, which are provided with absorbent properties.

High absorbent properties are necessary for the cloth to be wetted and retain a sufficiently high amount of the substance solution of the desired type (detergent, cosmetic and so on).

The above raw material fibres are generally hydroentangled or firmly bonded and are wetted with the solution of detergent and/or perfume, so that they can be packaged ready for use.

Environment considerations however would discourage using these products. As the material used to make the wipes is not biodegradable, they are not flushable, so that they should be disposed as solid rubbish. This is not practical in most cases.

WO2011019895 describes a nonwoven fabric comprising a nonwoven substrate, solid additives and a bonding material which is bonded to said nonwoven substrate.

WO2011009997 describes a nonwoven fabric comprising two external layers made of continous filaments of a nonwoven webs and a middle layer comprising a sheet material made from woodpulp fibres and thermoplastic fibres or filaments.

US2005/0106223 describes a trilaminated structure impregnated with a liquid emulsion.

An object of the present invention is therefore to provide a nonwoven fabric material that overcome the above disadvantages.

A further object of the invention is the process for preparing said nonwoven fabric material.

A further object of the invention are products, such as wipes and cleaning cloths, made of the nonwoven fabric material of the invention.

Further characteristics and the advantages of this invention will be better understood from the following detailed description of some embodiments thereof, which are provided by way of non-limiting examples wherein:
Figure 1 shows a schematic side view of a plant for manufacturing the invention nonwoven fabric material according to a first embodiment;
Figure 2 shows a schematic side view of a plant for manufacturing the invention nonwoven fabric material according to a second embodiment;
Figure 3 shows a schematic side view of a plant for manufacturing the invention nonwoven fabric material according to a third embodiment;
Figure 4 shows a schematic side view of a plant for manufacturing the invention nonwoven fabric material according to a fourth embodiment;
Figure 5 shows a schematic side view of a plant for manufacturing the invention nonwoven fabric material according to a fifth embodiment;
Figure 6 shows a schematic side view of a plant for manufacturing the invention nonwoven fabric material according to a sixth embodiment;
Figure 7 shows a graph reporting the results of the Tipping Tube test.

The nonwoven fabric material of the invention is a multi-layered material of claim 1.

The nonwoven fabric material of the invention is a dry material. The term "dry material" as used herein means that the material of the invention is manufactured according to technologies and methods at the dry state, i.e. that do not involve the use of water. In particular, no bonding through hydroentanglement is performed. The water content of the inventive material can be attributed to the amount of water that is normally contained in or absorbed from the environment by the materials as such.

The dry material of the invention has a water content of less than 10% by weight or less than 5% by weight.

The said water-absorbent material comprises or is made of cellulose pulp fibres. Preferably, the said cellulose pulp fibres have a length that may vary from substantially 0, i.e. cellulose powder, to 2.5 mm, preferably from 1 to 2 mm.

In one embodiment, the said water-soluble or water-dispersible polymer is selected from polyvinyl alcohol (PVA), carboxymethyl cellulose, Guar Gum, alginate (also called alginic acid), starch, a co-polyester and mixtures thereof.

Polyvinyl alcohol is a water-soluble, biodegradable synthetic polymer having a density of between 1.19 and 1.31 g/cc and a melting point of 230°C.

Carboxymethylcellulose is a cellulose derivative with carboxymethyl groups bound to some of the hydroxyl groups of the glucopyranose units. It is water-soluble and degradable.

Guar Gum is a galactomannan of natural origin, biodegradable.

Alginate is a water-dispersible anionic polysaccharide having a density of 1.601 g/cc.

Water-dispersible copolyesters are synthetic products developed by Eastman Chemical. They can be used in nonwoven applications, such as described in US 6,087,550. These polymers are commercially available.

The continuous filaments will preferably have a diameter between 0.5 dtex and 6.7 dtex, preferably a diameter of between 0.9 and 2.5 dtex, more preferably about 2 dtex.

The nonwoven fabric material of the invention comprises dry substances such as: dry substances for household or dry substances for personal care, or dry substances for topic medical use and/or microcapsules for slow releasing of one or more of such substances.

Examples of a substance for household application are: dry detergents and surfactants, waxes for ceramic or wooden floor, waxes for wood furniture surfaces, surface disinfectants, antibacterial, antiviral and/or antifungal products for household use, metal or wood polishing creams and cleaning formulations in general.

In one embodiment, the dry detergent is sodium alfa-olefin (C14-C16) sulphonate.

Examples of a substance for personal care are: formulations for personal hygiene and/or sanitization, skin creams or waxes, tanning creams, sunscreen formulations, insect repellent formulations, deodorants, perfumes, antibacterial, antiviral and/or antifungal formulations, make-up removing substances and cosmetic products in general.

Examples of a dry substance for topic medical use are: skin disinfectants, skin antibacterial, antiviral and/or antifungal substances, cicatrizing formulations and in general any drug formulation that can be administered by topical application, or sanitizing compositions for medical facilities, appliances or devices.

With the term "dry substance" it is intended herein a substance having a water content less than 10%, preferably less than 5%.

The nonwoven fabric material of the invention can also contain SAF (super-absorbent fibres) and/or SAP (super-absorbent polymers powder).

Super-absorbent polymers are commonly made from the polymerization of acrylic acid blended with sodium hydroxide in the presence of an initiator to form a poly-acrylic acid sodium salt. Other material are also used, such as polyacrylamide copolymer, ethylene maleic anhydride copolymer, cross-linked carboxymethyl cellulose, polyvinyl alcohols copolymers, cross-linked polyethylene oxides and starch-grafted copolymer of polyacrilonitrile and much others. They are normally made by suspension polymerization or solution polymerization. SAP and SAF are commercially available.

Several types of additives can also be added to the nonwoven fabric material, such as: lubricating additives to give smoothness and easy processability; antistatic additives to prevent damaging electrostatic currents that may degrade the product, or at worst, reduce the productivity of the machine; hydrophilic additives; anti-foam additives to avoid that foam may be generated upon use.

The nonwoven fabric material according to the invention typically has a weight/surface ratio (basis weight) ranging between 30 and 100 g/m², preferably more than 40 and less than 60 g/m².

The nonwoven fabric material of the invention is manufactured according to a process of claim 17.

The process can also comprise one or more steps of consolidation or pre-consolidation of the single layers of continuous filaments and/or of the multi-layered material before the one or more bonding step.

The process will comprise usually a final step of winding the nonwoven fabric material to make a fabric bobbin.

The said spunbond filaments can be produced in line with the formation of the nonwoven fabric material or alternatively are made on a separate production line.

The said spunbond filaments may be produced through extrusion by spinnerets of the above defined polymer materials so as to form continuous filaments. These filaments, on output from the spinnerets, are hit by a jet of compressed air that causes the elongation and the electrostatic charging thereof such as to cause a mutual repulsion causing them to fall randomly onto a conveyor belt. The continuous filament may be obtained by a spinning process by means of 1- to 5-orifices, preferably 2-3 orifices, spinner.

The pre-bonding consolidation of the single layers or of the multi-layered nonwoven fabric material can be accomplished by passing the layered material through two rollers of a compactor, embosser or calender.

It should be noted that by the term compactor or embosser is meant herein a device known per se, which has only the function of changing the surface of a nonwoven ply to obtain a slight consolidation (pre-consolidation) and in addition, in the case of the embosser, such as to form patterns, writings or drawings in relief. In other words, the compactor would have a pre-consolidation function, actually weak, whereas the embosser would have a preconsolidation and ornamental function, thereby increasing the thickness of the ply. On the contrary, the conventional calender, though being provided with a similar general structure, has the basic function to consolidate and partially bond the filaments composing the nonwoven while minimizing or at most maintaining the ply thickness being laid down.

One of the two rollers, in a conventional calendar, is engraved, i.e. it has ribs in the form of dots or dashes evenly alternating with grooves. In particular, the ribs have a height comprised between 0.4 and 0.6 mm, a free head with a contact surface for the filaments of 0.88 mm² and a distribution so that to cover 19-23% of the surface of the roller. It is to be noticed that said combination of features is responsible of a firm consolidation of the nonwoven ply.

In one embodiment, the nonwoven fabric material is a bonded web structure that comprises more than one web, preferably 3 to 9 webs. With a number of webs in the range specified above, a more isotropic textile structure, and accordingly a maximized spatial layout of the filaments is achieved, which results into a maximized filament-water contact surface. Thereby, the water droplets are adsorbed by the structure within the small spaces resulting from the random distribution of the filaments.

The step of bonding of the single layers or of the multi-layered material can be accomplished by means of a so called dry-laid process. Dry-laying procedure may comprise providing a web comprising 5 to 30 w% of a bicomponent fibre and passing it into a dryer wherein the material is subject to temperatures of between 120 and 200°C for a time generally comprised between 3 and 15 seconds. The high temperature melts the low-temperature melting polymer of the bicomponent fibres, thus establishing bonding points throughout the web. This process, known as air through bonding, is advantageous if a softer and thicker web structure is desired, especially if a side-by-side bicomponent fibre is used.

In a different embodiment, the single layers or the multi-layered material are bonded by a well-known chemical bonding process. In this case, the web structure is treated - for example by printing, powder or foam application - with a solid form of a latex polymer or a binder, in amounts ranging from 5 to 60 w%. The so treated web structure is then cured, for example by heat treatment. Suitable binders are selected from styrenebutadiene rubber, vinyl copolymers, vinyl acetate, styrenated or vinyl acrylates, polyvinylchloride. The binder formulation may comprise further ingredients, such as: surfactants (to improve binder adhesion, stability and ability to be converted into a foam); external crosslinkers; defoamers (to minimize foam in the process); repellent agents; salts (to impart low flame response properties and to convey antistatic properties); thickeners (to control rheology of the binder liquid); catalysts (to promote curing and cross-linking); acids and bases (to control pH of the binder); dyes and pigments; fillers (to reduce binder tack and to lower cost); optical brighteners (to increase whiteness); sewing aids (to provide lubrication).

In another embodiment, bonding of the single layers or of the multi-layered material is accomplished by means of a needle-punching process. The material is mechanically entangled by means of a multiplicity of barbed metal needles which mechanically move in a rapid reciprocating fashion forwards and backwards through the web, in a direction essentially perpendicular to the plane of the web. This movement of the barbed needles causes filaments and fibres within the web to become entangled with nearby filaments and fibres, making the web a coherent, strong structure.

According to the invention, it is convenient to slightly delay the water-solubility or water-dispersibility of the nonwoven fabric material to improve its handiness during use, when the material, in some applications, is wetted, without however impairing its flushability after use.

The nonwoven fabric material obtained as described above is treated with hydrophobic substances.

The material is impregnated with a solution of a derivative of a fatty acid in a non-aqueous organic solvent. In an exemplary embodiment, a solution of stearoyl chloride in toluene is used. Stearoyl chloride (CAS [112-76-5] is employed as a mixture of C18H35ClO and C16H31ClO.

This impregnation step comprises soaking the nonwoven fabric material into the fatty acid derivative solution for 5 to 60 sec and drying it at about 100°C for 5 to 30 minutes.

The nonwoven fabric material so treated will contain from 0.5 to 10 w%, preferably from 0.6 to 5 w%, of hydrophobic substance.

An experiment has been run to determine whether this treatment improves the resistance to water-solubility or water-dispersibility of the material (Tipping Tube test).

The wipe has been introduced in a plastic tube filled with 700 mL of water. The tube has been subjected to 240 circular rotations. The number of rotations has been recorded for three points:
1st end point: the samples are broken in 1x1 cm pieces;
2nd end point: the samples are broken in 5x5 mm pieces;
3rd end point: the samples are fully dispersed.

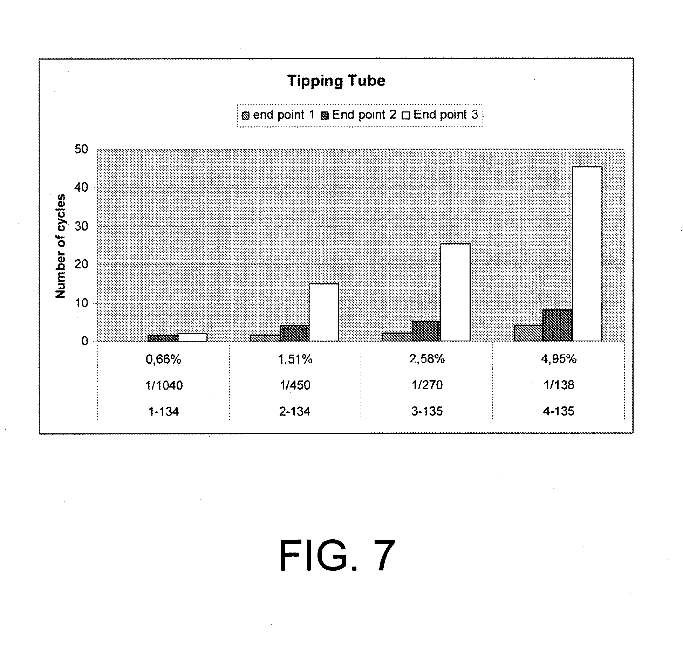

The results are reported in figure 7.

As one can see, the hydrophobic treatment increases significantly the resistance of the sample to the water-dispersion.

The process of the invention can also comprise one or more steps of adding to the single layers and/or to the nonwoven fabric material, before or after the bonding step, the additional substances defined above.

The single layers or the material can be impregnated with the said substance by any known method. In particular, delivery of the substance on the web by means of suitable nozzles can advantageously be used. For creams and waxes, hot melt delivery can advantageously be used, due to the high viscosity of such substance forms.

In an embodiment, the process comprises one or more of the following final steps, known per se, in order to increase or add additional characteristics to the end product: coloring or finishing of a chemical nature as the anti-pilling treatment and the hydrophilic treatment, antistatic treatment, improvement of flame proof properties, substantially mechanical treatments such as napping, sanforizing, emerizing.

The invention will be now further described by means of exemplary embodiments, with reference to the attached drawings. The drawings do not show the stations for adding the above defined additional substances, however the said stations are conventional and can be positioned at any point along the production line, preferably before the last bonding step.

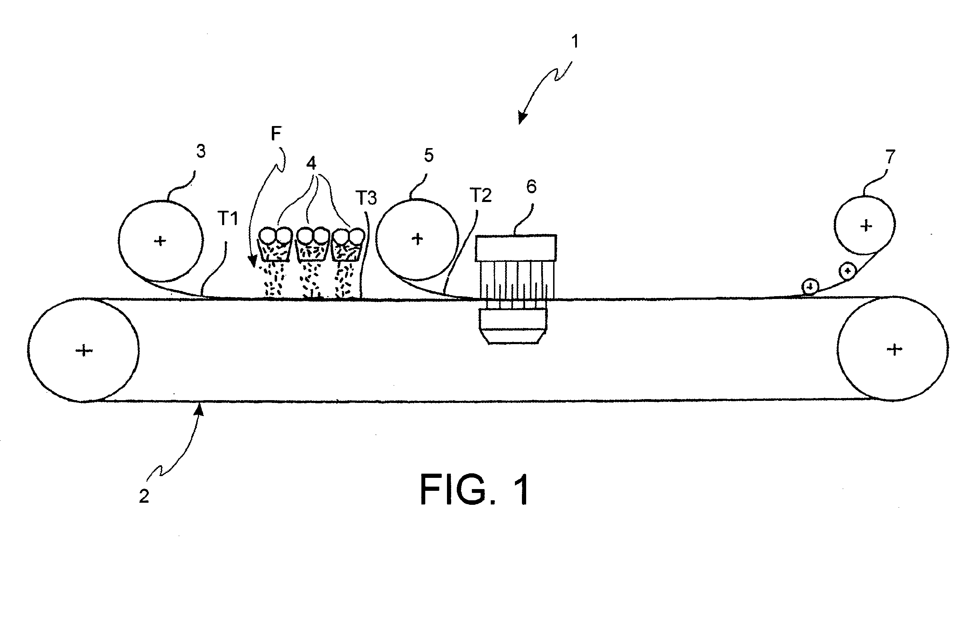

In figure 1 it is shown a first embodiment of a production line 1 for manufacturing the inventive material.

On a transporting means 2, typically a conveyor belt, a layer T1 of consolidated spunbond continuous filaments is unwound from a feeding bobbin 3.

Subsequently, on the said layer T1 water-absorbent fibres F, typically cellulose pulp fibres, are laid through suitable dispensing means 4 to form a layer T3.

After the deposition of said water-absorbent fibres, a second layer T2 of consolidated spunbond continuous filaments is unwound and laid on the previous layers, after which the tri-layer material is passed through dry bonding means 6 (in the example of figure 1, needle-punching means) .

Finally, the bonded nonwoven fabric material is wound on a receiving bobbin 7.

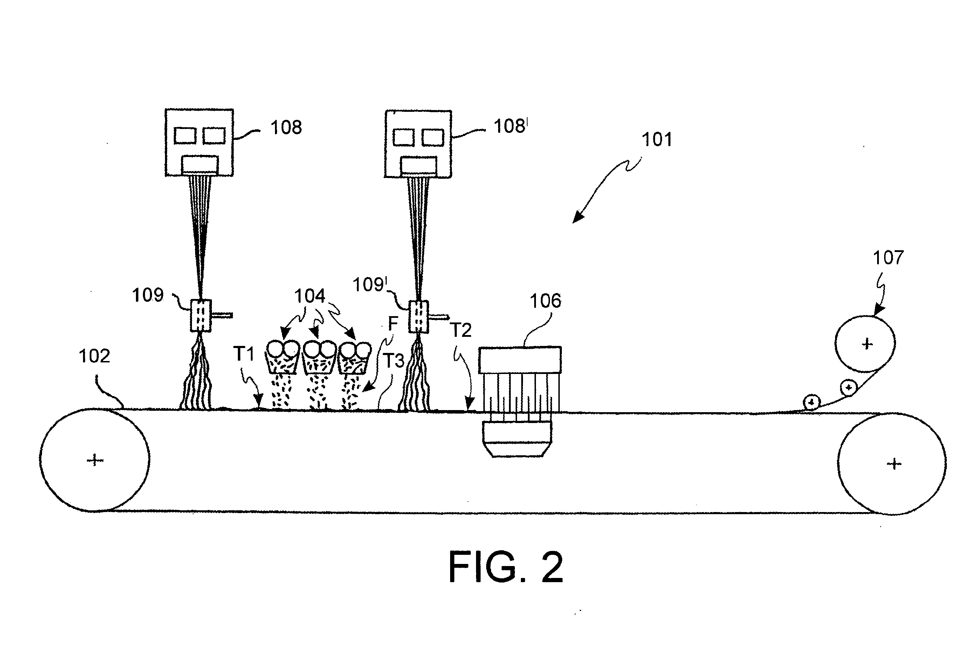

Figure 2 shows a different embodiment, wherein a trilayered material is obtained by producing in line the spunbond continuous filaments.

On a transporting means 102, typically a conveyor belt, a layer T1 of spunbond continuous filaments is laid after having been extruded through a spinneret 108 coupled to a suction fan 109, according to a conventional technique.

Subsequently, on the said layer T1 water-absorbent fibres F, typically cellulose pulp fibres, are laid through suitable dispensing means 104 to form a layer T3.

After the deposition of said water-absorbent fibres, a second layer T2 of spunbond continuous filaments is extruded from a second spinneret 108'-suction fan 109' and laid over the previous layers.

The tri-layer material is then passed through dry bonding means 106 (in the example of figure 2, needle-punching means) and is finally wound on a receiving bobbin 107.

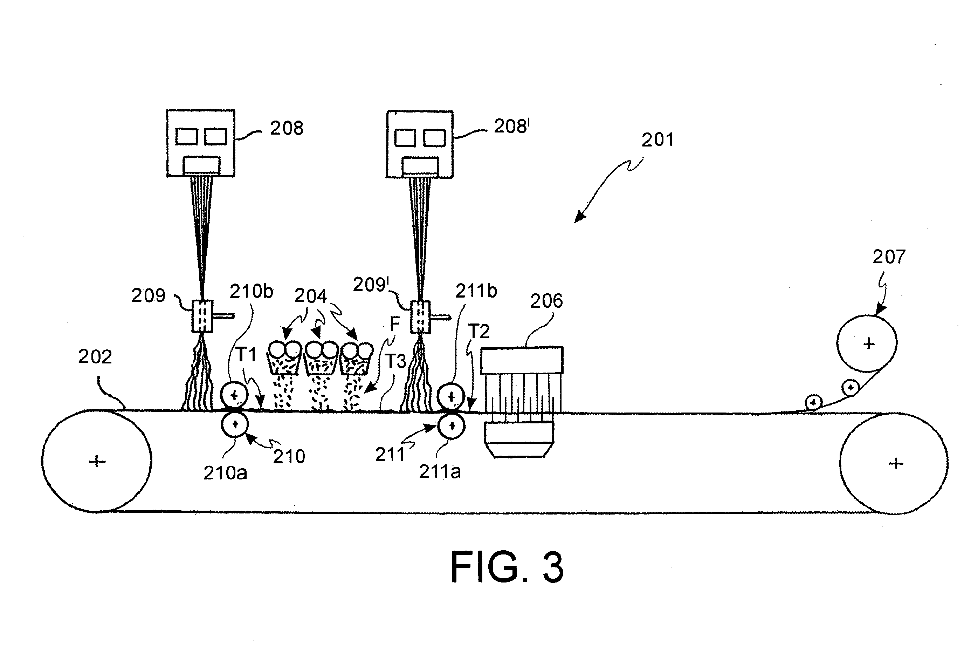

Figure 3 shows another embodiment that differs from the embodiment of figure 2 in that, after the deposition of both the first layer T1 and the second layer T2 of spunbond continuous filament, consolidation or pre-consolidation means are provided.

On a transporting means 202, typically a conveyor belt, a layer T1 of spunbond continuous filaments is laid after having been extruded through a spinneret 208 coupled to a suction fan 209, according to a conventional technique.

The said layer T1 of spunbond continuous filaments, supported by the transporting means 202, is passed through first consolidation or pre-consolidation means 210, comprising two rollers 210a, 210b coupled in such a way to allow the said layer T1 to be pressed therebetween. These consolidation or pre-consolidation means 210 are a compactor, an embosser or a calender, as defined above.

Subsequently, on the said layer T1 water-absorbent fibres F, typically cellulose pulp fibres, are laid through suitable dispensing means 204 to form a layer T3.

After the deposition of said water-absorbent fibres, a second layer T2 of spunbond continuous filaments is extruded from a second spinneret 208'-suction fan 209' and laid over the previous layers.

The said layered material, supported by the transporting means 202, is passed through second consolidation or pre-consolidation means 211, comprising two rollers 211a, 211b coupled in such a way to allow the said layered material to be pressed therebetween. These consolidation or pre-consolidation means 211 are a compactor, an embosser or a calender, as defined above.

The tri-layer material is then passed through dry bonding means 206 (in the example of figure 3, needle-punching means) and is finally wound on a receiving bobbin 207.

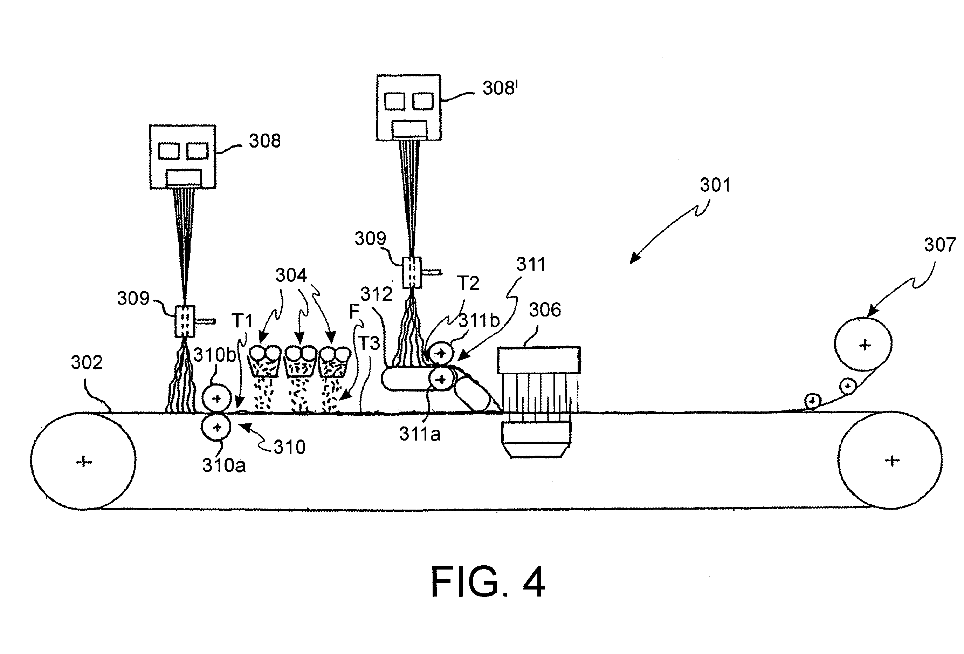

The further embodiment shown in figure 4 differs from the embodiment in figure 3 in that the second layer of spunbond continuous filaments is independently consolidated or pre-consolidated before laying it over the other layers of nonwoven material.

On a transporting means 302, typically a conveyor belt, a layer T1 of spunbond continuous filaments is laid after having been extruded through a spinneret 308 coupled to a suction fan 309, according to a conventional technique.

The said layer T1 of spunbond continuous filaments, supported by the transporting means 302, is passed through first consolidation or pre-consolidation means 310, comprising two rollers 310a, 310b coupled in such a way to allow the said layer T1 to be pressed therebetween. These consolidation or pre-consolidation means 310 are a compactor, an embosser or a calender, as defined above.

Subsequently, on the said layer T1 water-absorbent fibres F, typically cellulose pulp fibres, are laid through suitable dispensing means 304 to form a layer T3.

After the deposition of said water-absorbent fibres, a second layer T2 of spunbond continuous filaments is extruded from a second spinneret 308'-suction fan 309' and laid over transporting means 312 positioned above the transporting means 302 carrying the already deposited layers T1 and T3. The said transporting means 312 are coupled to consolidation or pre-consolidation means 311, similar to the ones described above and comprising two rollers 311a, 311b of a compactor, embosser or calender. The second layer T2 of spunbond continuous filaments is thus consolidated or pre-consolidated and then laid over the previous layers passing therebelow.

The layered material so obtained, supported by the transporting means 302, is then passed through dry bonding means 306 (in the example of figure 4, needle-punching means) and is finally wound on a receiving bobbin 307.

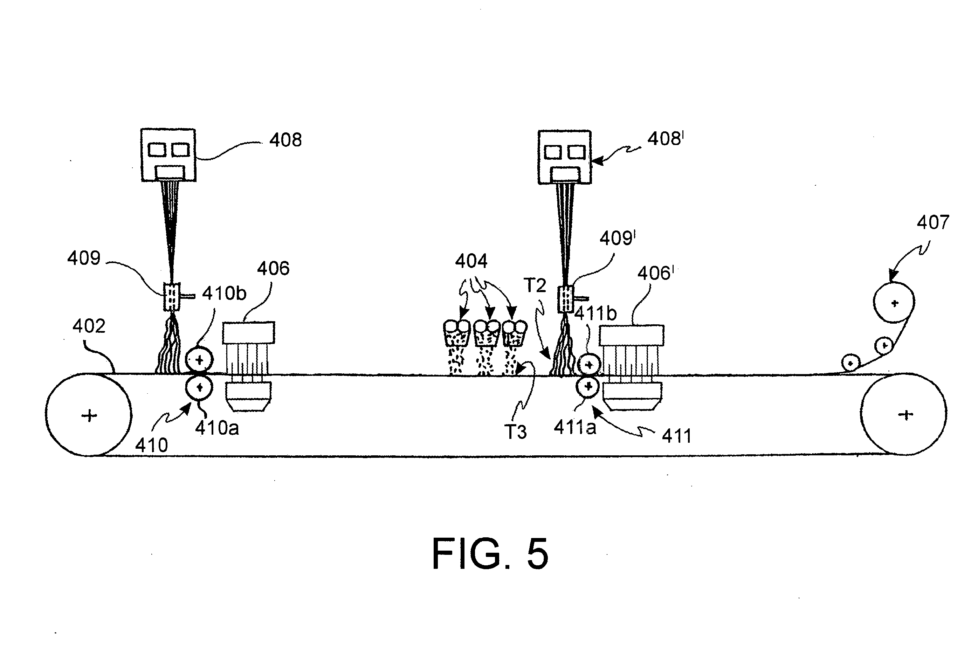

The embodiment shown in figure 5 differs from the embodiment of figure 3 in that the first layer T1 of spunbond continuous filaments is bonded before the deposition of the other layers.

On a transporting means 402, typically a conveyor belt, a layer T1 of spunbond continuous filaments is laid after having been extruded through a spinneret 408 coupled to a suction fan 409, according to a conventional technique.

The said layer T1 of spunbond continuous filaments, supported by the transporting means 402, is passed through first consolidation or pre-consolidation means 410, comprising two rollers 410a, 410b coupled in such a way to allow the said layer T1 to be pressed therebetween. These consolidation or pre-consolidation means 410 are a compactor, an embosser or a calender, as defined above.

After having been consolidated or pre-consolidated, the layer T1 is bonded by passing it through dry bonding means 406 (in the example of figure 5, needle-punching means) .

Subsequently, on the said layer T1 water-absorbent fibres F, typically cellulose pulp fibres, are laid through suitable dispensing means 404 to form a layer T3.

After the deposition of said water-absorbent fibres, a second layer T2 of spunbond continuous filaments is extruded from a second spinneret 408'-suction fan 409' and laid over the previous layers.

The said layered material, supported by the transporting means 402, is passed through second consolidation or pre-consolidation means 411, comprising two rollers 411a, 411b coupled in such a way to allow the said layered material to be pressed therebetween. These consolidation or pre-consolidation means 411 are a compactor, an embosser or a calender, as defined above.

The tri-layer material is then passed through second dry bonding means 406' (in the example of figure 5, needle-punching means) and is finally wound on a receiving bobbin 407.

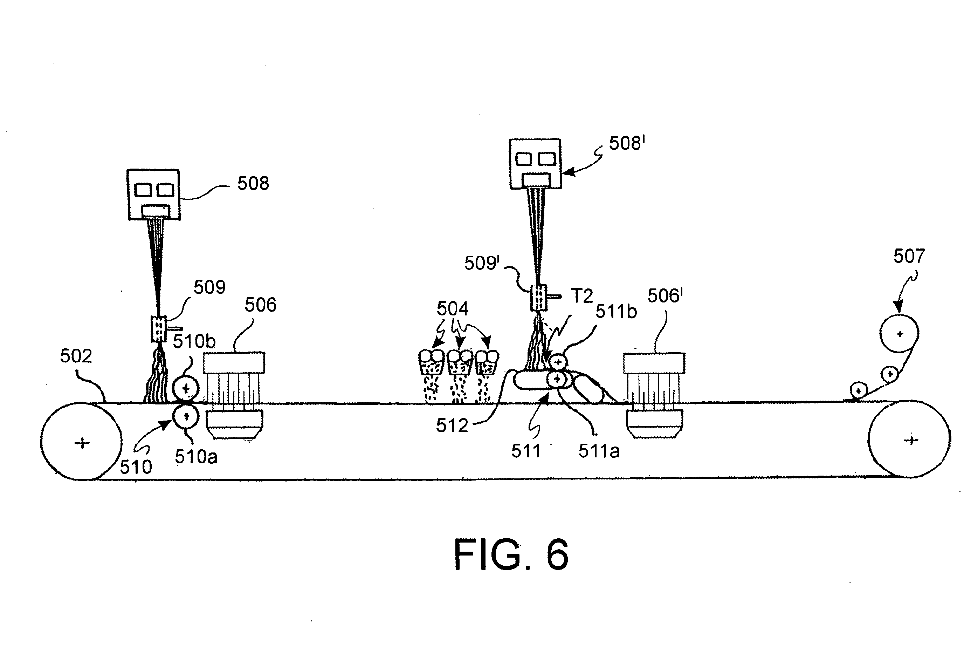

The embodiment shown in figure 6 differs from the embodiment of figure 5 in that the first layer T1 of spunbond continuous filaments is bonded before the deposition of the other layers.

On a transporting means 502, typically a conveyor belt, a layer T1 of spunbond continuous filaments is laid after having been extruded through a spinneret 508 coupled to a suction fan 509, according to a conventional technique.

The said layer T1 of spunbond continuous filaments, supported by the transporting means 502, is passed through first consolidation or pre-consolidation means 510, comprising two rollers 510a, 510b coupled in such a way to allow the said layer T1 to be pressed therebetween. These consolidation or pre-consolidation means 510 are a compactor, an embosser or a calender, as defined above.

After having been consolidated or pre-consolidated, the layer T1 is bonded by passing it through dry bonding means 506 (in the example of figure 6, needle-punching means).

Subsequently, on the said layer T1 water-absorbent fibres F, typically cellulose pulp fibres, are laid through suitable dispensing means 504 to form a layer T3.

After the deposition of said water-absorbent fibres, a second layer T2 of spunbond continuous filaments is extruded from a second spinneret 508'-suction fan 509' and laid over transporting means 512 positioned above the transporting means 502 carrying the already deposited layers T1 and T3. The said transporting means 512 are coupled to consolidation or pre-consolidation means 511, similar to the ones described above and comprising two rollers 511a, 511b of a compactor, embosser or calender. The second layer T2 of spunbond continuous filaments is thus consolidated or pre-consolidated and then laid over the previous layers passing therebelow.

The layered material so obtained, supported by the transporting means 502 is then passed through second dry bonding means 506' (in the example of figure 6, needle-punching means) and is finally wound on a receiving bobbin 507.

The invention further comprises products for household or personal care made of the nonwoven fabric material defined above. Examples of such products are cloths, rags, wipes and similar fabrics.

These products are dry or substantially dry, therefore they must be wetted before use. Once the product has been used, it can be disposed and flushed without inconvenient, as the materials of which it is made are water-soluble or water dispersible and generally biodegradable.

In an embodiment the products are water-soluble or water-dispersible at room temperature, i.e. generally at or below 25°C.

## Claims

1. A nonwoven fabric material comprising two external layers made of continuous filaments of a water-soluble or water-dispersible polymer and at least one internal layer of a water-absorbent, flushable material, **characterized in that** it has a water content of less than 10% by weight or less than 5% by weight and **in that** it also comprises dry substances for household or dry substances for personal care, or dry substances for topic medical use and/or microcapsules for slow releasing of one or more of such substances, wherein said water-absorbent material is made of cellulose pulp fibres, wherein said nonwoven fabric material comprises a hydrophobic substance which is a derivative of a fatty acid.

2. The nonwoven fabric material of claim 1, that is a dry trilayered material comprising one internal layer of cellulose pulp fibres, one upper layer of said continuous filaments of a water-soluble or water-dispersible polymer and one lower layer of said continuous filaments of a water-soluble or water-dispersible polymer.

3. The nonwoven fabric material of claim 1, wherein the said cellulose pulp fibres have a length that vary from substantially 0, i.e. cellulose powder, to 2.5 mm, preferably from 1 to 2 mm.

4. The nonwoven fabric material according to any one of claims 1 to 3, wherein the said water-soluble or water-dispersible polymer is selected from polyvinyl alcohol (PVA), carboxymethyl cellulose, Guar Gum, alginate, starch, a water-dispersible co-polyester and mixtures thereof.

5. The nonwoven fabric material according to any one of claims 1 to 4, wherein the said continuous filaments have a diameter between 0.5 dtex and 6.7 dtex, preferably a diameter of between 0.9 and 2.5 dtex, more preferably about 2 dtex.

6. The nonwoven fabric material of any claim 1 to 5, wherein the said substances for household application are selected from dry detergents and surfactants, waxes for ceramic or wooden floors, waxes for wood furniture surfaces, surface disinfectants, antibacterial, antiviral and/or antifungal products for household use, metal or wood polishing creams and cleaning formulations in general.

7. The nonwoven fabric material of claim 6, wherein the dry detergent is sodium alfa-olefin (C14-C16) sulphonate.

8. The nonwoven fabric material of any claim 1 to 5, wherein the said substance for personal care are selected from formulations for personal hygiene and/or sanitization, skin creams or waxes, tanning creams, sunscreen formulations, insect repellent formulations, deodorants, perfumes, antibacterial, antiviral and/or antifungal formulations, make-up removing substances and cosmetic products in general.

9. The nonwoven fabric material of any claim 1 to 5, wherein the said substances for topic medical use are selected from skin disinfectants, skin antibacterial, antiviral and/or antifungal substances, cicatrizing formulations and in general any drug formulation that can be administered by topical application, or sanitizing compositions for medical facilities, appliances or devices.

10. The nonwoven fabric material according to any one of claims 1 to 9, comprising super-absorbent fibres and/or super-absorbent polymer powder.

11. The nonwoven fabric material of claim 10, wherein the said super-absorbent polymers are selected from poly-acrylic acid sodium salt, polyacrylamide copolymer, ethylene maleic anhydride copolymer, cross-linked carboxymethyl cellulose, polyvinyl alcohols copolymers, cross-linked polyethylene oxides and starch-grafted copolymer of polyacrilonitrile.

12. The nonwoven fabric material according to any one of claims 1 to 11, comprising one or more additive selected from: lubricating additives; antistatic additives; hydrophilic additives; anti-foam additives.

13. The nonwoven fabric material according to any one of claims 1 to 12, having a weight/surface ratio (basis weight) ranging between 30 and 100 g/m², or more than 40 and less than 60 g/m².

14. The nonwoven fabric material according to any one of claims 1 to 13, which is water-soluble or water-dispersible at room temperature.

15. The nonwoven fabric material according to any one of claims 1 to 14, wherein said hydrophobic substance which is a derivative of a fatty acid is in an amount comprised between 0.5 to 10 w% or 0.6 to 5 w% acid.

16. The nonwoven fabric material according to claim 15, wherein the derivative of a fatty acid is stearoyl chloride.

17. A process for manufacturing the nonwoven fabric material according to any one of claims 1 to 16, comprising the following steps:
a) Laying a first layer of spunbond filaments made of a water-soluble or water-dispersible polymer;
b) Laying, on the layer of step a), a layer of water-absorbable fibres;
c) Laying another layer of said spunbond filaments;
d) Bonding the single layers and/or the multi-layered material at the dry state, to provide one or more bonding step, wherein the one or more bonding step is performed in such a way that the so obtained nonwoven fabric material has a water content of less than 10% by weight or less than 5% by weight;
e) Impregnating the nonwoven fabric material of step d) with dry substances for household or dry substances for personal care or dry substances for topic medical use and/or microcapsules for slow releasing of one or more of such substances;
f) Soaking the nonwoven fabric material into a fatty acid derivative solution.

18. The process of claim 17, comprising one or more steps of consolidation or pre-consolidation of the single layers of continuous filaments and/or of the multi-layered material before the one or more bonding step.

19. The process of claim 17 or 18, wherein the said spunbond filaments are produced in line with the formation of the said nonwoven fabric material or alternatively are made on a separate production line.

20. The process of any one of claims 17 to 19, wherein the said consolidation or pre-consolidation of the single layers and/or of the multi-layered nonwoven fabric material can be accomplished by passing the layered material through two rollers of a compactor, embosser or calendar.

21. The process according to any one of claims 17 to 20, wherein the said step of bonding of the single layers and/or of the multi-layered material is accomplished by means of a dry-laid process.

22. The process according to any one of claims 17 to 20, wherein the said step of bonding of the single layers and/or of the multi-layered material is accomplished by means of a chemical bonding process.

23. The process according to any one of claims 17 to 20, wherein the said step of bonding of the single layers and/or of the multi-layered material is accomplished by means of a needle-punching process.

24. The process according to any one of claims 17 to 23, comprising one or more steps of adding to the single layers and/or to the nonwoven fabric material, before or after the said one or more bonding step, one or more substances as defined in any one of claims 6 to 12.

25. The process according to any one of claims 17 to 24, comprising one or more of the following steps:coloring or finishing of a chemical nature as the anti-pilling treatment and the hydrophilic treatment, antistatic treatment, improvement of flame proof properties, substantially mechanical treatments such as napping, sanforizing, emerizing.

26. The process according to any one of Claims 17 to 25, wherein the step of soaking the said nonwoven fabric material into the fatty acid derivative solution is performed with a solution of a derivative of a fatty acid in a non-aqueous organic solvent.

27. The process according to claim 27, wherein the step of soaking the said nonwoven fabric material into the fatty acid derivative solution is performed with a toluene solution of stearoyl chloride.

28. Products for household or personal care, comprising or made of a nonwoven fabric material according to any one of claims 1 to 16.

29. Products according to claim 28, being a cloth, a rag or a wipe.

## Patentansprüche

1. Vliesmaterial, umfassend zwei äußere Schichten, hergestellt aus Endlosfasern aus einem wasserlöslichen oder wasserdispergierbaren Polymer, und mindestens eine innere Schicht aus einem wasserabsorbierenden, spülbaren Material, **dadurch gekennzeichnet, dass** es einen Wassergehalt von weniger als 10 Gew.-% oder weniger als 5 Gew.- % aufweist, und dass es auch Trockensubstanzen für Haushalt oder Trockensubstanzen für Körperpflege oder Trockensubstanzen für topische medizinische Anwendung und/oder Mikrokapseln für langsame Freisetzung einer oder mehrerer solcher Substanzen umfasst, worin das wasserabsorbierende Material aus Cellulosepulpefasern hergestellt ist, worin das Vliesmaterial eine hydrophobe Substanz umfasst, die ein Derivat einer Fettsäure ist.

2. Vliesmaterial nach Anspruch 1, das trockenes dreischichtiges Material ist, umfassend eine innere Schicht aus Cellulosepulpefasern, eine obere Schicht aus den Endlosfasern aus einem wasserlöslichen oder wasserdispergierbaren Polymer und eine untere Schicht aus den Endlosfasern aus einem wasserlöslichen oder wasserdispergierbaren Polymer.

3. Vliesmaterial nach Anspruch 1, worin die Cellulosepulpefasern eine Länge aufweisen, die von im Wesentlichen 0, d.h. Cellulosepulver, bis 2,5, vorzugsweise von 1 bis 2 mm variiert.

4. Vliesmaterial nach einem der vorhergehenden Ansprüche 1 bis 3, worin das wasserlösliche oder wasserdispergierbare Polymer ausgewählt ist aus Polyvinylalkohol (PVA), Caboxymethylcellulose, Guargummi, Alginat, Stärke, einem wasserlöslichen Co-Polyester und Gemischen davon.

5. Vliesmaterial nach einem der vorhergehenden Ansprüche 1 bis 4, worin die Endlosfilamente einen Durchmesser zwischen 0,5 dtex und 6,7 dtex, vorzugsweise einen Durchmesser zwischen 0,9 und 2,5 dtex, mehr bevorzugt etwa 2 dtex haben.

6. Vliesmaterial nach einem der vorhergehenden Ansprüche 1 bis 5, worin die Substanzen für Haushaltsanwendung ausgewählt sind aus Trockendetergenzien und oberflächenaktiven Mitteln, Wachsen für Keramik- oder Holzböden, Wachsen für Holzmöbeloberflächen, Oberflächendesinfektionsmitteln, antibakteriellen, antiviralen und/oder antifungiellen Produkten für Haushaltsanwendung, Metall- oder Holzpoliturcremes und Reinigungsformulierungen im Allgemeinen.

7. Vliesmaterial nach Anspruch 6, worin das Trockendetergens Natriumalpha-olefin (C₁₄-C₁₆)-sulfonat ist.

8. Vliesmaterial nach einem der vorhergehenden Ansprüche 1 bis 5, worin die Substanzen für Körperpflege ausgewählt sind aus Formulierungen für Körperhygiene und/oder Sanitisierung, Hautcremes oder Wachsen, Bräunungscremes, Sonnenschutzformulierungen, Insektenschutzmittelformulierungen, Deodorants, Parfums, antibakteriellen, antiviralen und/oder antifungiellen Formulierungen, Make-up-Entfernungsmitteln und kosmetischen Produkten im Allgemeinen.

9. Vliesmaterial nach einem der vorhergehenden Ansprüche 1 bis 5, worin die Substanzen für topische medizinische Anwendung ausgewählt sind aus Hautdesinfektionsmitteln, antibakteriellem Hautbehandlungsmittel, antiviralen und/oder antifungiellen Substanzen, Narbenbildingsformulierungen und im Allgemeinen einer beliebigen Arzneimittelformulierung, die durch topische Anwendung verabreicht werden kann, oder Sanitisierungszusammensetzungen für medizinische Anlagen, Einrichtungen oder Geräte.

10. Vliesmaterial nach einem der vorhergehenden Ansprüche 1 bis 9, umfassend superabsorbierende Fasern und/oder superabsorbierendes Polymerpulver.

11. Vliesmaterial nach einem der vorhergehenden Ansprüche 1 bis 10, worin die superabsorbierenden Polymere ausgewählt sind aus Polyacrylsäure-Natriumsalz, Polyacrylamid-Copolymer, Ethylen-Maleinsäureanhydrid-Copolymer, quervernetzte Carboxymethylcellulose, Copolymere von Polyvinylalkoholen, quervernetzte Polyethylenoxide und Stärke-gepropftes Copolymer von Polyacrylnitril.

12. Vliesmaterial nach einem der vorhergehenden Ansprüche 1 bis 11, umfassend ein oder mehrere Additive, ausgewählt aus: Gleitmitteladditive, Antistatikadditive; hydrophile Additive, Antischaumadditive.

13. Vliesmaterial nach einem der vorhergehenden Ansprüche 1 bis 12 mit einem Verhältnis von Gewicht/Oberfläche (Flächengewicht) im Bereich zwischen 30 und 100 g/m² oder mehr als 40 und kleiner als 60 g/m².

14. Vliesmaterial nach einem der vorhergehenden Ansprüche 1 bis 13, das wasserlöslich oder wasserdispergierbar bei Raumtemperatur ist.

15. Vliesmaterial nach einem der vorhergehenden Ansprüche 1 bis 14, worin die hydrophobe Substanz, die ein Derivat einer Fettsäure ist, in einer Menge von 0,5 bis 10 Gew.-% oder 0,6 bis 5 Gew.-% umfasst ist.

16. Vliesmaterial nach Anspruch 15, worin das Derivat einer Fettsäure Stearoylchlorid ist.

17. Verfahren zum Herstellen des Vliesmaterials nach einem der Ansprüche 1 bis 16, umfassend die folgenden Schritte:
a. Auslegen einer ersten Schicht aus Spinnvliesfilamenten, die hergestellt sind aus wasserlöslichem oder wasserdispergierbarem Polymer;
b. Auflegen auf der Schicht aus Schritt a) einer Schicht aus wasserabsorbierenden Fasern;
c. Auflegen einer anderen Schicht der Spinnvliesfilamente;
d. Verbinden der einzelnen Schichten und oder des mehrschichtigen Materials im trockenen Zustand, um ein oder mehrere Bindungsschritte vorzusehen, worin der eine oder die mehreren Bindungsschritte so durchgeführt werden, dass des erhaltene Vliesmaterial einen Wassergehalt von weniger als 10 Gew.-% oder weniger als 5 Gew.-% aufweist;
e. Imprägnieren des Vliesmaterials aus Schritt d) mit Trockensubstanzen für den Haushalt oder Trockensubstanzen für Körperpflege oder Trockensubstanzen für topische medizinische Anwendung und/oder Mikrokapseln für langsame Freisetzung einer oder mehrerer solcher Substanzen;
f. Tränken des Vliesmaterials mit einer Fettsäurederivatlösung.

18. Verfahren nach Anspruch 17, umfassend einen oder mehrere Schritte der Verfestigung oder Vorverfestigung der einzelnen Schichten aus Endlosfilamenten und/oder des mehrschichtigen Materials vor dem einen oder den mehreren Verbindungsschritten.

19. Verfahren nach Anspruch 17 oder 18, worin die Spinnfilamente zusammen mit der Bildung des Vliesmaterials hergestellt werden oder alternativ auf einer getrennten Fertigungsanlage hergestellt werden.

20. Verfahren nach einem der Ansprüche 17 bis 19, worin die Verfestigung oder Vorverfestigung der einzelnen Schichten und/oder des mehrschichtigen Vliesmaterials durchgeführt werden kann durch durchführen des geschichteten Materials durch zwei Walzen eines Kompaktors, einen Prägekalander oder Kalander.

21. Verfahren nach einem der Ansprüche 17 bis 20, worin der Verbindungsschritt der einzelnen Schichten und/oder des Mehrschichtmaterials mit einem Trockenauflegeverfahren durchgeführt wird.

22. Verfahren nach einem der Ansprüche 17 bis 20, worin der Verbindungsschritt der einzelnen Schichten und/oder des Mehrschichtmaterials mit einem chemischen Verbindungsverfahren durchgeführt wird.

23. Verfahren nach einem der Ansprüche 17 bis 20, worin der Verbindungsschritt der einzelnen Schichten und/oder des Mehrschichtmaterials mit einem Vernadelungsverfahren durchgeführt wird.

24. Verfahren nach einem der Ansprüche 17 bis 23, umfassend einen oder mehrere Schritte des Hinzufügens einer oder mehrerer oben in einem der Ansprüche 6 bis 12 definierten Substanzen zu den einzelnen Schichten oder zu dem Vliesmaterial vor oder nach dem einen oder den mehreren Verbindungsschritten.

25. Verfahren nach einem der Ansprüche 17 bis 24, umfassend einen oder mehrere der folgenden Schritte: Färben oder Endbehandeln einer chemischen Natur, wie die Anti-Pilling-Behandlung und die hydrophile Behandlung, Antistatikbehandlung, Verbesserung der Flammschutzeigenschaften, wesentliche mechanische Behandlungen, wie etwa Aufrauen, Sanforisieren, Schmirgeln.

26. Verfahren nach einem der Ansprüche 17 bis 25, worin der Schritt des Tränkens des Vliesmaterials mit der Fettsäurederivatlösung mit einer Lösung eines Derivats einer Fettsäure in einem nichtwässrigen organischen Lösungsmittel durchgeführt wird.

27. Verfahren nach Anspruch 26, worin der Schritt des Tränkens des Vliesmaterials mit der Fettsäurederivatlösung mit einer Toluollösung von Stearylchlorid durchgeführt wird.

28. Produkte für Haushalts- Körperpflegeanwendung, umfassend oder hergestellt aus einem Vliesmaterial nach einem der Ansprüche 1 bis 16.

29. Produkte nach Anspruch 28, die ein Tuch, Lappen oder Wischtuch sind.

## Revendications

1. Matériau d'étoffe non tissée comprenant deux couches externes faites de filaments continus d'un polymère soluble dans l'eau ou dispersible dans l'eau et au moins une couche interne d'un matériau absorbant l'eau et pouvant être jeté dans les toilettes, **caractérisé en ce qu'**il a une teneur en eau inférieure à 10 % en poids ou inférieure à 5 % en poids et **en ce qu'**il comprend aussi des substances sèches à usage domestique ou des substances sèches pour hygiène corporelle, ou des substances sèches pour usage médical topique et/ou des microcapsules pour libération lente d'une ou plusieurs de ces substances, dans lequel ledit matériau absorbant l'eau est fait de fibres de pâte de cellulose, lequel matériau d'étoffe non tissée comprend une substance hydrophobe qui est un dérivé d'un acide gras.

2. Matériau d'étoffe non tissée selon la revendication 1, qui est un matériau sec à trois couches comprenant une couche interne en fibres de pâte de cellulose, une couche supérieure en lesdits filaments continus d'un polymère soluble dans l'eau ou dispersible dans l'eau, et une couche inférieure en lesdits filaments continus d'un polymère soluble dans l'eau ou dispersible dans l'eau.

3. Matériau d'étoffe non tissée selon la revendication 1, dans lequel lesdites fibres de pâte de cellulose ont une longueur qui varie de pratiquement 0, c'est-à-dire de la poudre de cellulose, à 2,5 mm, de préférence de 1 à 2 mm.

4. Matériau d'étoffe non tissée selon l'une quelconque des revendications 1 à 3, dans lequel ledit polymère soluble dans l'eau ou dispersible dans l'eau est choisi parmi le poly(alcool vinylique) (PVA), la carboxyméthylcellulose, la gomme de Guar, l'alginate, l'amidon, un copolyester dispersible dans l'eau, et leurs mélanges.

5. Matériau d'étoffe non tissée selon l'une quelconque des revendications 1 à 4, dans lequel lesdits filaments continus ont un diamètre compris entre 0,5 dtex et 6,7 dtex, de préférence un diamètre compris entre 0,9 et 2,5 dtex, mieux encore d'environ 2 dtex.

6. Matériau d'étoffe non tissée selon l'une quelconque des revendications 1 à 5, dans lequel lesdites substances pour application domestique sont choisies parmi les tensioactifs et les détergents secs, les cires pour sols en céramique ou en bois, les cires pour surfaces d'ameublement en bois, les désinfectants de surface, les produits antibactériens, antiviraux et/ou antifongique à usage domestique, les crèmes de polissage pour métal ou bois, et les formulations nettoyantes en général.

7. Matériau d'étoffe non tissée selon la revendication 6, dans lequel le détergent sec est une α-oléfinesulfonate de sodium en C₁₄ à C₁₆.

8. Matériau d'étoffe non tissée selon l'une quelconque des revendications 1 à 5, dans lequel ladite substance pour hygiène corporelle est choisie parmi les formulations pour hygiène corporelle et/ou aseptisantes, les cires ou crèmes pour la peau, les crèmes bronzantes, les formulations d'écran solaire, les formulations insectifuges, les déodorants, les parfums, les formulations antibactériennes, antivirales et/ou antifongiques, les substances démaquillantes et les produits cosmétiques en général.

9. Matériau d'étoffe non tissée selon l'une quelconque des revendications 1 à 5, dans lequel lesdites substances à usage médical topique sont choisies parmi les désinfectants cutanés, les substances antibactériennes, antivirales et/ou antifongiques pour la peau, les formulations cicatrisantes et en général n'importe quelle formulation de médicament qui peut être administrée par application topique, et les compositions aseptisantes pour installations, appareillages ou dispositifs médicaux.

10. Matériau d'étoffe non tissée selon l'une quelconque des revendications 1 à 9, comprenant des fibres superabsorbantes et/ou une poudre polymère superabsorbante.

11. Matériau d'étoffe non tissée selon la revendication 10, dans lequel lesdits polymères superabsorbants sont choisis parmi le poly(acrylate de sodium), un copolymère de polyacrylamide, un copolymère d'éthylène-anhydride maléique, la carboxyméthylcellulose réticulée, les copolymères de poly(alcool vinylique), les poly(oxydes d'éthylène) réticulés et les copolymères de polyacrylonitrile greffés d'amidon.

12. Matériau d'étoffe non tissée selon l'une quelconque des revendications 1 à 11, comprenant un ou plusieurs additifs choisis parmi : les additifs lubrifiants ; les additifs antistatiques ; les additifs hydrophiles ; les additifs anti-mousse.

13. Matériau d'étoffe non tissée selon l'une quelconque des revendications 1 à 12, ayant un rapport poids/surface (sur une base en poids) située dans la plage comprise entre 30 et 100 g/m², ou supérieur à 40 et inférieur à 60 g/m².

14. Matériau d'étoffe non tissée selon l'une quelconque des revendications 1 à 13, qui est soluble dans l'eau ou dispersible dans l'eau à la température ambiante.

15. Matériau d'étoffe non tissée selon l'une quelconque des revendications 1 à 14, dans lequel ladite substance hydrophobe qui est un dérivé d'un acide gras est présente en une quantité comprise entre 0,5 et 10 % en poids ou entre 0,6 et 5 % en poids.

16. Matériau d'étoffe non tissée selon la revendication 15, dans lequel le dérivé d'un acide gras est le chlorure de stéaroyle.

17. Procédé pour fabriquer le matériau d'étoffe non tissée selon l'une quelconque des revendications 1 à 16, comprenant les étapes suivantes :
a) déposition d'une première couche de filaments filés-liés faits en un polymère soluble dans l'eau ou dispersible dans l'eau ;
b) déposition, sur la couche de l'étape a), d'une couche de fibres pouvant absorber l'eau ;
c) déposition d'une autre couche desdits filaments filés-liés ;
d) collage des monocouches et/ou du matériau multicouche à l'état sec, en réalisant une ou plusieurs étapes de collage, la ou les étapes de collage étant mises en oeuvre de façon que le matériau d'étoffe non tissée ainsi obtenu ait une teneur en eau inférieure à 10 % en poids ou inférieure à 5 % en poids ;
e) imprégnation du matériau d'étoffe non tissée de l'étape d) avec des substances sèches à usage domestique ou des substances sèches pour hygiène corporelle ou des substances sèches pour usage médical topique et/ou des microcapsules pour libération lente d'une ou plusieurs de ces substances ;
f) trempage du matériau d'étoffe non tissée dans une solution d'un dérivé d'acide gras.

18. Procédé selon la revendication 17, comprenant une ou plusieurs étapes de consolidation ou de pré-consolidation des monocouches de filaments continus et/ou du matériau multicouche avant la ou les étapes de collage.

19. Procédé selon la revendication 17 ou 18, dans lequel lesdits filaments filés liés sont produits en ligne avec la formation dudit matériau d'étoffe non tissée, ou en variante sont fabriqués sur une ligne de production séparée.

20. Procédé selon l'une quelconque des revendications 17 à 19, dans lequel ladite consolidation ou pré-consolidation des monocouches et/ou du matériau d'étoffe non tissée multicouche peut être accomplie par passage du matériau stratifié entre deux rouleaux d'un compacteur, d'une gaufreuse ou d'une calandre.

21. Procédé selon l'une quelconque des revendications 17 à 20, dans lequel ladite étape de collage des monocouches et/ou du matériau multicouche est accomplie au moyen d'un procédé par voie sèche.

22. Procédé selon l'une quelconque des revendications 17 à 20, dans lequel ladite étape de collage des monocouches et/ou du matériau multicouche est accomplie au moyen d'un procédé de collage chimique.

23. Procédé selon l'une quelconque des revendications 17 à 20, dans lequel ladite étape de collage des monocouches et/ou du matériau multicouche est accomplie au moyen d'un procédé d'aiguilletage.

24. Procédé selon l'une quelconque des revendications 17 à 23, comprenant une ou plusieurs étapes d'addition aux monocouches et/ou au matériau d'étoffe non tissée, avant ou après ladite ou lesdites étapes de collage, d'une ou plusieurs substances telles que définies dans l'une quelconque des revendications 6 à 12.

25. Procédé selon l'une quelconque des revendications 17 à 24, comprenant une ou plusieurs des étapes suivantes : coloration ou finissage de nature chimique sous forme de traitement anti-boulochage et de traitement hydrophile, traitement antistatique, amélioration des propriétés ignifuges, traitements sensiblement mécaniques tels que duvetage, sanforisage, polissage.

26. Procédé selon l'une quelconque des revendications 17 à 25, dans lequel l'étape de trempage dudit matériau d'étoffe non tissée dans la solution d'un dérivé d'acide gras est effectuée avec une solution d'un dérivé d'un acide gras dans un solvant organique non aqueux.

27. Procédé selon la revendication 27, dans lequel l'étape de trempage dudit matériau d'étoffe non tissée dans la solution d'un dérivé d'acide gras est effectuée avec une solution toluénique de chlorure de stéaroyle.

28. Produits à usage domestique ou pour hygiène corporelle, comprenant ou faits d'un matériau d'étoffe non tissée selon l'une quelconque des revendications 1 à 16.

29. Produits selon la revendication 28, qui sont l'un parmi un torchon, un chiffon et une lingette.
